(19) Europäisches Patentamt
European Patent Office
Office européen des brevets

(11) Publication number: **0 053 479**
**B1**

(12) # EUROPEAN PATENT SPECIFICATION

(45) Date of publication of patent specification: **13.05.87**

(51) Int. Cl.$^4$: **A 61 B 5/02**

(21) Application number: **81305584.5**

(22) Date of filing: **25.11.81**

(54) **Acceleration pulse wave meter.**

(30) Priority: **29.11.80 JP 168396/80**

(43) Date of publication of application:
**09.06.82 Bulletin 82/23**

(45) Publication of the grant of the patent:
**13.05.87 Bulletin 87/20**

(84) Designated Contracting States:
**DE FR GB**

(56) References cited:
**CH-A- 547 088**
**DE-A-1 466 900**
**US-A-4 105 021**
**US-A-4 137 910**
**US-A-4 144 879**
**US-A-4 260 951**

(73) Proprietor: **Osanai, Hiroshi**
**Miyamaedaira Green Heights 17-403**
**Mukogaoka 430 Takatsu-ku**
**Kawasaki-shi Kanagawa-ken (JP)**

(72) Inventor: **Osanai, Hiroshi**
**Miyamaedaira Green Heights 17-403**
**Mukogaoka 430 Takatsu-ku**
**Kawasaki-shi Kanagawa-ken (JP)**

(74) Representative: **Brock, Peter William et al**
**Michael Burnside & Partners 2 Serjeants' Inn**
**Fleet Street**
**London EC4Y 1HL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 053 479 B1

Courier Press, Leamington Spa, England.

**Description**

This invention relates to the evaluation of the quality of circulation of blood.

In the past, the functional examination of circulatory organs of the body has relied upon an electrocardiogram and blood pressure measurements. However, apart from the blood pressure measurements, opinion is divided on the manner of proper interpretation of an electrocardiogram and problems are involved so that electrocardiograms are therefore difficult to understand. Also, an electrocardiogram, for example an exercise electrocardiogram, can seize a change in a function in a special case and a given organic change can appear, but the electrocardiogram cannot give basis for judgement of the quality of blood circulation to assist in preventing a change in a disease in the future and to provide data necessary to recover a bodily function.

Recently it has become appreciated that a person who does not always have high blood pressure can often be suffering from infarction or subarachnoidal haemorrhage and the mutual relationship therebetween is unknown, and accordingly, there is a demand for new means of examination to evaluate blood circulation.

On the other hand a plethysmograph has been used as one of various examination means for the circulatory function. That is, the pulse wave which is an increase or decrease in the volume of blood within a capillary blood vessel consequent upon a heart beat is mechanically or electrically seized as a fluctuation. A sensor for a plethysmograph utilises a change in physical volume of a finger or the like, a change in impedance or a change in light transmissibility. Parts of the body to be detected include not only peripheral organs but various other parts of the body.

Clinically speaking, the peripheral blood stream is checked to thereby to use it for examination of abnormality of the aortic valve, degree of arteriosclerosis, propagation time, effect of blood vessel expanding agent, adjustment of vascular exercise, and transition of intoxication of pregnancy. This method however is deficient in reliability because it is liable to be affected by the temperature, tension of the mind, drugs, respiration, etc. and in addition, delusions in judgement can occur due to the obscurity of shape of the pulse waveforms given by the conventional plethysmograph. For this reason, other examination methods had to be referred to while avoiding formation of judgements only by such plethysmography. Plethysmography is therefore rarely utilised these days, and a plethysmograph is often merely used as a heart beat signal of a heat beat meter.

It is therefore an object of the present invention to provide for seizing the characteristics of an individual person to be examined, from the information given by plethysmography, in such a manner as easily to extract information which is highly related to the clinical results of circulatory dysfunction.

CH—A—547088 describes a plethysmograph which comprises a pick-up adapted to detect variations in the blood content of a finger-tip consequent upon a heat beat and to provide a first signal representative of said variations, circuit means for twice-differentiating said first signal with respect to time, and means for displaying or recording said twice-differentiated signal in such a manner in relation to a magnitude reference axis as to indicate the relative magnitudes of valleys and peaks of the waveform of said twice-differentiated signal.

In one aspect, the present invention provides the use of a twice-differentiated plethysmographic signal to provide an evaluation of the quality of circulation of blood in a blood capillary bed between an artery and a vein of a finger-tip, said signal having been obtained by means of a plethysmograph, which is known per se (see CH—A—547088 summarized above) and which comprises a pick-up adapted to detect variations in the blood content of said finger-tip consequent upon a heart beat and to provide a first signal representative of said variations, circuit means for twice-differentiating said first signal with respect to time, and means for displaying or recording said twice-differentiated signal in such a manner in relation to a magnitude reference axis (Figures 48 and 49) as to indicate the relative magnitudes of at least the first and second valleys (B, D) and the second peak (C) of the waveform of said twice-differentiated signal, characterised in that said magnitudes of said valleys (B, D) and peak (C) are compared with predetermined values of said magnitudes to provide a quantitative evaluation of said blood circulation quality.

US—A—4105021 describes automatic diagnostic apparatus comprising:

a pick-up adapted to receive a finger therein, to detect a predetermined physiological condition in said finger and to provide a first signal representative of said detected physiological condition;

means utilizing a diagnostic algorithm to obtain from said first signal diagnostic data related to said predetermined physiological condition; and

means for displaying said diagnostic data.

The physiological condition detected by the apparatus described in US—A—4105021 is blood pressure, and the diagnostic data displayed are the systolic and diastolic pressures. The blood flow condition in the finger is simultaneously monitored by a flow monitor, which can be a plethysmograph, to provide a signal for the signal analysing circuitry of the utilizing means.

In another aspect, the present invention provides said automatic diagnostic apparatus characterised in that:

said pick-up is a plethysmographic pick-up adapted to detect variations in the blood content of a finger-tip consequent upon a heart beat and to provide said first signal representative of said variations (known per se);

circuit means is provided for twice-differentiating said first signal with respect to time (known per se);

said utilizing means comprises means for comparing the magnitude of the waveform of said twice-differentiated signal in relation to a magnitude reference axis (Figures 48 and 49), said diagnostic algorithm being utilized to compare the relative magnitudes of at least the first and second valleys (B, D) and the second peak (C) of the waveform of said twice-differentiated signal with a plurality of predetermined standard waveforms (Figure 48) to provide an evaluation of the quality of circulation of blood in a blood capillary bed between an artery and a vein of said finger-tip.

Said evaluation has been found to be representative of the quality of fine circulation of blood in the body as a whole.

In carrying out the present invention, a finger-tip is selected as the body part to be examined for the reasons as follows. The pulse wave in the finger-tip serves as the responsive means at a location where a blood capillary bed is developed and the blood content is relatively large, in seizing a movement of blood from a peripheral artery to a vein, and therefore the finger-tip can be said to be the optimum place to seize the condition of blood circulation.

In addition, the finger-apex is always exposed and is accessible to a detection portion of the examination apparatus, which construction may be advantageously designed simply.

Incidentally, causes influencing on the pulse wave of the finger-apex include three points as follows:

(1) Cause of artery side
- a. Heart beat
- b. Distribution of blood to the upper limbs
- c. Elasticity of artery system

In *a*, *b* and *c*, it functions as the continuous force.

(2) Cause of blood capillaries

Content of blood determined by density and broadness, Temperature

(3) Cause of vein side

a. Diastole<br>
b. Elasticity of vein } Pressure functions as negative pressure

Since the pulse wave of finger-apex records a change in contained-blood volume, blood extruded by the heart beat arrives at the blood capillaries after a delay of a given time. Since the blood is present also in the vein side, the blood does not flow uniformly through the blood capillaries to the vein but remains in the artery side by a portion in excess of the range of blood received by the vein. However, extrusion pressure on the artery side is well above the volume of blood moving to the vein, the cycle which reduces as the blood capillaries of the finger-apex expand is repeated. This transition is seized by the pulse wave of the finger-apex. However, relatively continuous pressure is applied to the artery system due to the heart beat, elasticity of the artery system or the like whereas in the vein system, the vein wall is relatively thin and the elasticity is scarce in addition to the influence thereon by the diastole, as a consequence of which a change in pressure is delicate. Accordingly, the waveform of the pulse wave of the finger-apex is varied while being affected by the delicate change in pressure of the vein system. The change in content of blood can be known from the ordinary pulse wave, and the speed of increase or decrease in content of blood can be seen by the waveform of a linear differentiation. However, information on the change in content of blood corresponding to the change in pressure of the vein system cannot be well seized unless it is differentiated once again into an acceleration curve.

Considering now the quadratic differentiation of the volume of blood V contained in the blood capillaries as a model, V may be written as the product of a sectional area S in vessel of a blood-contained portion and the length of said contained portion as in the following equation:

$$V = l \cdot S \qquad (1)$$

Differentiating V, l, S as the fluent at time t, then

$$\frac{dV}{dt} = l \cdot \frac{dS}{dt} + \frac{dl}{dt} \cdot S \qquad (2)$$

Further differentiating them once again at time t, then

$$\frac{d^2V}{dt^2} = \frac{dl}{dt} \cdot \frac{dS}{dt} + l \cdot \frac{d^2S}{dt^2} + \frac{d^2l}{dt^2} \cdot S + \frac{dl}{dt} \cdot \frac{dS}{dt}$$

$$= 2 \cdot \frac{dl}{dt} \cdot \frac{dS}{dt} + l \cdot \frac{d^2S}{dt^2} + \frac{d^2l}{dt^2} \cdot S \qquad (3)$$

In the right side, the first term represents the product of the flow speed of blood and the expansion speed of the blood capillary, the second term represents the product of the content of blood and the expansion speed of the blood capillaries, and the third term represents the product of the blood flow acceleration and the sectional area of the blood contained portion. It is here considered that the flow speed and flow acceleration are affected by pressure of residual blood on the peripheral vein side, and the expansion speed of the sectional area and expansion acceleration are affected by the residual blood in the peripheral vein and the expansion resistance of the blood capillaries.

The quadratic differentiated figure illustrates the change acceleration of the content of blood in the capillary vessel. In other words, it illustrates a change in speed produced in response to a delicate change in vein pressure. In the past, the delicate change in vein pressure has not been seized by the peripheral vein, and also, the quadratic differentiated figure of the pulse wave has not been accounted for sufficiently. However, it seems that the waveform has a given pattern and the recognition of the pattern can provide data by which the quality of the blood circulation may be judged with considerable accuracy.

Referring now to the quadratic differentiated figure of the pulse wave shown in Figure 3, when heart beat blood having a blood pressure of 20—30 mmHg (1mmHg=133,3 Pa) arrives at a small artery and flows into the blood capillaries, the volume of said blood is in excess of that delivered towards the vein through the capillary vessel and as a consequence, the change acceleration of the content in the capillary vessel rapidly increases (O→A). Such an increase rapidly reduces subsequently since the blood in the capillary vessel is fed to the vein (A→B), and in case that the vein is filled with blood to some extent, the blood from the vein stays in the capillary vessel once again with the result that the acceleration of the change in contained blood volume in the capillary vessel tends to increase (B→C). For the same reason as mentioned previously, fluctuation is repeated in which the acceleration then reduces slightly (C→D), further increases (D→E) and then reduces (E→F), after which point G, the change remains unconspicuous. This can be seen also in the waveform shown in Figure 2 which is normally seized as a pulse wave, but it has been heretofore merely seized as a minute point of inflection and thus analysis has been difficult.

In accordance with the present invention, movement of blood from the artery to the vein through the blood capillaries is not seized as a given flow but seized as a fluctuation corresponding to the change in mutual pressure to read data for judgement of the quality of the blood stream from the shape of the fluctuation thereby definitely extracting the characteristics therefor.

Figure 1 is a block diagram showing the entire construction;

Figure 2 (a, b, c and d) shows pulse waves;

Figure 3 is a quadratic differentiated figure (acceleration pulse wave curve) in accordance with the present invention;

Figure 4 is a schematic view showing a blood flow in blood capillaries;

Figure 5, 6, 7, 8, 9, 10, 11, 12, 13, 14 and 15 are respectively views showing pulse waves, changed speed thereof, and changed acceleration thereof for eleven persons to be examined;

Figure 16 is a comparative view for the presence of a difference in the acceleration pulse wave curve by way of fingers;

Figure 17, 18, 19, 20, 21, 22, 23, 24, 25, 26, 27, 28, 29, 30, 31, 32, 33, 34, 35, 36, 37, 38, 39, 40, 41, 42, and 43 are respectively views showing changes in acceleration pulse wave curve where exercise is imposed on 27 persons to be examined;

Figures 44, 45, 46 (a) (b) and 47 are respectively views showing the acceleration pulse wave curve of persons having past anamnesis;

Figure 48 is a pattern classifying view of the acceleration pulse wave curve;

Figure 49 is a conceptual view for counting marks relating to the blood flow of individuals from a single quadratic differentiated figure;

Figure 50 (a, b and c) is a bar graph showing the distribution of marks by age of an examined group;

Figure 51 (a, b and c) is a similar bar graph showing the distribution of marks of a group of male and female exercisers and unexercisers in university students;

Figures 52, 53 and 55 are respectively block diagrams showing modifications;

Figure 54 (a and b) is a comparative view between the quadratic differentiated and cubic differentiated figures; and

Figure 56 is a perspective view of a robot doctor to which the present invention is applied.

The construction of a plethysmography in accordance with the present invention will now be described in detail by way of one embodiment.

Embodiment 1

As shown in a block diagram of Figure 1, the plethysmography comprises a finger-apex pulse wave pickup 5 provided with an opening 2 into which a finger 1 is inserted, a light source 3 and a photoelectric element 4, a preamplifier 6, an OP amplifier 7, a characteristic extract circuit 8 and a pen-written oscillograph 9. In the embodiment shown in Figure 1, three kinds of graphs, i.e., a pulse wave, a once-differentiated value of the pulse wave and a twice-differentiated value thereof, but the function of the characteristic extract circuit 8 is not limited thereto as will be described later. In such a structure as described above, pulse wave waveforms and varying acceleration waveforms in which said pulse waveforms are differentiated twice continuously at time t by the characteristic extract circuit 8 were observed with the result that waveforms as in Figure 2 (a, b, c, d) and Figure 3 were obtained. For carrying out a quadratic differentiation, two stages of analog differentiating circuit employing a CR circuit were employed. The axis of abscissa indicates the time t, and the axis of ordinate indicates the volume of blood contained in a capillary vessel (hereinafter referred to as "the contained-blood volume" and the varying acceleration of the same.

In Figure 2(a), the peak 10 is constantly delayed in time corresponding to the heart beat whereas the peak 11 subsequently appeared is considered to correspond to the contained-blood volume on the vein side. There are cases such as being sharply recognizable as in Figure 2 (b), recognizable at a point where the contained-blood volume decreases as in Figure 2 (c), dimly recognizable slightly delayed from the peak 10 as in Figure 2 (a), and hardly recognizable as in Figure 2 (d), which cases are not constant, resulting in a painstaking of making judgment thereof which leads to the cause in which the pulse wave curve is of no practical value, as previously described.

Observing now waveforms (Figure 3) of the varying acceleration of the contained-blood volume of the blood capillaries in accordance with the present invention, there is normally recognized three peaks (A, C, E) and two valleys (B, D) therebetween, and as compared with a number of clinical examples, the state of blood movement from artery to vein through the blood capillaries can be distinguishably known from the height of peak C and depth of valley D relative to the reference line X—X'. It is observed that the peak C is high and the valley D is shallow for a young and high-spirited person who is in the good condition of blood circulation. And, for a middle and advanced aged person who is poor in circulation of the blood, sometimes the peak C does not only reach the reference line but is hardly recognized and such a change can be recognized that the valley D becomes extremely deepened much more than the valley B. It has become obvious that information representative of such poor circulation of the blood is obtained.

The difference between the above-mentioned conventional pulse wave and the acceleration pulse wave obtained by the plethysmography in accordance with the present invention will be explained with reference to actual examples shown in Figures 5, 6, 7, 8, 9, 10, 11, 12, 13, 14 and 15. These figures respectively show the measured results obtained from the age of 73 male, 64 male, 40 male, 31 female, 38 female, 26 male, 36 male, 52 male, 39 male, 26 male, and 23 male in order from Figure 5, the upper column being representative of the conventional pulse wave, the middle column the curve differentiated at time t, and the lower column the quadrative differentiated figure further differentiated once again at time t. It is hardly possible to discuss the difference between individuals from the pulse wave in the upper column and is difficult to clarify the difference even from the waveforms in the middle column. The quadrative differentiated figure in the lower column is the curve having a given pattern divided into several peaks and valleys different from the waveforms in the upper and lower columns and it is possible to say something or other of individual difference with respect to the relative height of peak and relative depth of valley.

These curves in the upper, middle and lower columns are concerned with a transition of the content of blood in the blood capillaries. The upper column indicates the transition by time of the content of blood in the blood capillaries in response to movement of heart beat, the middle column indicates the transition of speed of increase or decrease in the content of blood, and the lower column indicates the speed or acceleration in change of said speed. These curves all indicate the relative movement but the difference supposed depending on individuals and ages is most plainly indicated by the acceleration pulse wave curve in the lower column.

In case of one and the same person, there is not recognized a difference by fingers. That is, in Figure 16 (I, II, III, IV, V), the quadrative differentiated figures were compared between fingers from the first finger to the fifth finger, and as a result, only the first finger showed unifitness of a sensor and the second to fifth fingers mostly showed the same pattern. It is to be understood that unless the finger-apex excessively gets cold to minimize the circulation of blood, in case of one and the same person, a pattern peculiar to said person is represented irrespective of broadness of the finger.

Since the acceleration pulse wave curve is a reflex of the state of a blood flow in the capillary vessel, it also shows the form peculiar to the individual. For example, it is considered that if one takes a running for a long period of time, he can maintain his good blood flow condition for a long period of time and if one takes a continuous training, he can maintain his good blood flow condition. Loads or exercises such as various preparatory gymnastics, and runnings are imposed on an examined group given in Tables 1—1 and 1—2 to observe a change of the acceleration pulse wave curve in their rest conditions, which examples are illustrated in Figures 17, 18, 19, 20, 21, 22, 23, 24, 25, 26, 27, 28, 29, 30, 31, 32, 33, 34, 35, 36, 37, 38, 39, 40, 41, 42 and 43.

TABLE 1—1

| Measured date of acceleration pulse wave and status of load | | 17 | 18 | 19 | 20 | 21 | 22 | 23 | 24 | 25 | 26 | 27 | 28 | 29 |
|---|---|---|---|---|---|---|---|---|---|---|---|---|---|---|
| Fig. | | 17 | 18 | 19 | 20 | 21 | 22 | 23 | 24 | 25 | 26 | 27 | 28 | 29 |
| Sex distinction ♂ | | ○ | ○ | ○ | | | | | | | | | | |
| 〃 ♀ | | | | | ○ | ○ | ○ | ○ | ○ | ○ | ○ | ○ | ○ | ○ |
| Age | | 46 | 47 | 60 | 34 | 60 | 39 | 41 | 51 | 52 | 53 | 53 | 54 | 53 |
| Blood pressure | | 152/102 | 145/111 | 167/94 | 113/70 | 133/70 | 161/108 | 145/101 | 179/117 | 107/69 | 149/95 | 149/79 | 115/74 | 126/79 |
| Rest pulse/min. | | 77 | 101 | 69 | 78 | 67 | 67 | 77 | 72 | 59 | 66 | 63 | 59 | 63 |
| '80.8.22 | Measurement | ○ | ○ | ○ | ○ | ○ | ○ | ○ | ○ | ○ | ○ | ○ | ○ | ○ |
| | Load | / | / | / | / | / | / | / | / | / | / | / | / | / |
| '80.8.24 | Measurement | ○ | ○ | ○ | ○ | ○ | ○ | ○ | ○ | ○ | ○ | ○ | ○ | ○ |
| | Load | 1 | 10′×2 | 10′ | 1 | 10′×2 | 60′ | 1 | 40′ | 2/3 | 30′ | 10′×3 | / | 2/3 |
| '80.8.25 | Measurement | ○ | ○ | ○ | ○ | ○ | ○ | ○ | ○ | ○ | ○ | ○ | ○ | ○ |
| | Load | 120′ | 30′ | 10′ | 1 | 25′ | 4/5 | 60′ | 1 | 1/3 | 1 | 20′ | 10′×2 | 2/3 |
| '80.8.29 | Measurement | ○ | ○ | ○ | ○ | ○ | ○ | ○ | ○ | ○ | ○ | ○ | ○ | ○ |
| | Load | / | / | / | / | / | / | / | / | / | / | / | / | / |

TABLE 1—2

| Measured date of acceleration pulse wave and status of load | Fig. | 30 | 31 | 32 | 33 | 34 | 35 | 36 | 37 | 38 | 39 | 40 | 41 | 42 | 43 |
|---|---|---|---|---|---|---|---|---|---|---|---|---|---|---|---|
| | Sex distinction ♂ | | | | | | | | | | | | | | |
| | 〃 ♀ | ○ | ○ | ○ | ○ | ○ | ○ | ○ | ○ | ○ | ○ | ○ | ○ | ○ | ○ |
| | Age | 59 | 57 | 60 | 52 | 39 | 53 | 30 | 40 | 33 | 39 | 49 | 52 | 54 | 54 |
| | Blood pressure | 134/90 | 99/78 | 115/76 | 140/81 | 166/93 | 150/91 | 97/72 | 115/72 | 104/70 | 140/88 | 164/68 | 139/76 | 139/74 | 122/80 |
| | Rest pulse/min. | 65 | 69 | 73 | 74 | 85 | 81 | 60 | 55 | 69 | 68 | 107 | 75 | 78 | 73 |
| '80.8.22 | Measurement | ○ | ○ | ○ | ○ | ○ | ○ | ○ | ○ | ○ | ○ | ○ | ○ | ○ | ○ |
| | Load | / | / | / | / | / | / | / | / | / | / | / | / | / | / |
| '80.8.24 | Measurement | ○ | ○ | ○ | ○ | / | / | ○ | ○ | / | / | / | / | / | / |
| | Load | / | 10′×3 | 2/3 | 20′ | 25′ | 10′×2 | / | / | 1 | 10′×3 | 40′ | 10′×3 | 25′ | 25′ |
| '80.8.25 | Measurement | ○ | ○ | ○ | ○ | ○ | ○ | ○ | ○ | / | / | / | / | / | / |
| | Load | 1 | 25′ | 2/3 | 25′ | 1 | 1 | / | / | / | / | 2/3 | 25′ | 20′ | 25′ |
| '80.8.29 | Measurement | ○ | ○ | ○ | ○ | ○ | ○ | / | / | ○ | ○ | ○ | ○ | ○ | ○ |
| | Load | / | / | / | / | / | / | / | / | / | / | / | / | / | / |

In the Tables, loads 1, 4/5, 2/3, and 1/3 are abridgements of one round lake Yamanaka, 4/5 round, 2/3 round and 1/3 round, respectively. Others indicate the running time and frequencies.

These cases are varied according to the age, sex distinction, volume and time of training or running, conditions of daily life and so on, but some show their change, return to their original conditions or further improvement. The acceleration pulse wave curves for four persons with other anamnesis are illustrated in Figures 44, 45, 46 (a) (b), and 47 (a) (b). In Figure 44, in case of a certain person of age 50, 1973: angina pectoris, 1975: myocardial infarction, 1979: start of training, 1979 Aug.: discovery of myoma uteria, 1979 Nov.: operation of the same, Jun. 6, 1980: measurement of acceleration pulse wave (Figure 44), and thereafter loss of contact with her, probably died. In Figure 45, in case of a further certain person of age 45, May 1977: operation of myoma uteria, Apr. 1980: start of training, Apr. 4, 1980: measurement of acceleration pulse wave (Figure 45), the trend of improvement as compared with the case of Figure 44 is recognized. In Figure 46, in case of a certain person of age 56, April 1960: angina pectoris, Apr. 1976: start of training, Apr. 4, 1980: measurement of acceleration pulse wave (Figure 46 (a)). July 1, 1980: measurement of acceleration pulse wave (Figure 46 (b)), the trend of improvement is not recognized between both figures, remaining on the same level and thereafter loss of contact with her. She was probably dead. In Figure 47, in case of a certain person of age 58, 1969: subarachnoidal hemorrhage, Sept. 1976: start of training, Apr. 4, 1980: measurement of acceleration pulse wave (Figure 47 (a)). June 6, 1980: measurement of acceleration pulse wave (Figure 47 (b)), the trend of improvement is recognized between both figures and it was assured also from other diagnostic results that she is on the road of recovery.

Next, how to read the acceleration pulse wave curve will be described.

Pattern analysis of acceleration pulse was curve (1)

The quadrative differentiated figure of the pulse wave usually illustrates the peaks and valleys at A, B, C, D and E as shown in Figure 48, from various cases so far discussed. From the degree of the depth of valley B and valley D, there is divided into three relations, i.e., $b>d$, $b\fallingdotseq d$, and $b<d$, and there can be roughly divided into three from the point that the height of the peak C is higher or lower than the reference line X—X'.

The pattern in P—I is recognized in a young and high-spirited person who is good in circulation of the blood, and the pattern in P—II is recognized in a young person but often in a middle and advanced aged person who is relatively high-spirited.

The pattern in P—III is recognized rather in a person of tachycardia.

The pattern in Q—I—III is recognized rather in a person who has somewhat high blood pressure and is the pattern which is considered that the contained-blood volume of vein slightly increases with the result that the circulation of blood tends to be somewhat poor.

A person representative of the pattern in R—I—III often is high in blood pressure, and this pattern is considered to be a pattern representative of a poor blood flow in the capillary vessel resulting from an increase in pressure of vein.

However, these patterns are not always related, from experience, to blood pressure at that time but represent the quality of circulation of the blood. At least types of Q and R need sufficient care and if such conditions continue, dangerous hindrance of circulation would result, in which case, an improvement through training, running and so on is desirable in consideration thereof.

Pattern analysis of acceleration pulse wave curve (II)

As shown in Figure 49, the acceleration pulse wave curve has the peaks and valleys at A, B, C, D, E, and F, which among them is featurized by the height C of point C relative to the reference line X—X' and the depth d of point D relative to the reference line X—X'. For example, in Figure 50 (a), an advanced aged group over 60 years old was selected as examined persons from participants in a training and swimming class-room in Tama Sports Clubhouse and those in Hachioji Athletic Clubhouse, and these 121 persons were examined to show how many persons score the point C in what point by the age provided that the depth b from the reference line to point B is divided into four parts so that O point is given for those above the reference line, one point for those directly below the reference line, and two, three, four and five points for those therebelow. As the age advances, there are many persons who have higher marks, and the average marks by the age tends to gradually increase.

The same is true for point D (Figure 50 (b)). Marks of C+d added together also show a similar tendency (Figure 50 (c)). It is assumed that those who have obtained higher marks are poor in circulation of the blood in termms of individuals as well as groups. This can be applied to the individuals to represent the quality of blood circulation of a specific individual for use as data for judgement.

Further, Figure 51 (a) (b) (c) are similar distribution views for students in a certain university. The results were that in (c), and exerciser was good whereas in (d), an unexerciser was good. However, there are a difference between young persons and advanced aged persons and a difference between individuals. Thus, it is one of advantages of the present invention that the aforesaid difference is clarified to watch the development thereby obtaining more positive data.

Embodiment 2

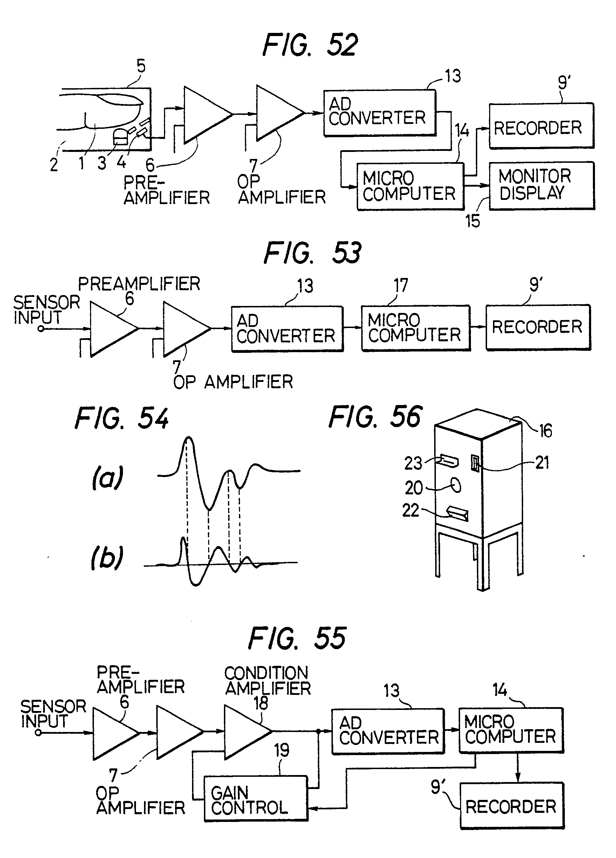

In the aforementioned embodiment 1, the CR analog system has been employed in the quadratic differentiated circuit for the characteristic extract. However, in place of such system, an acceleration plethysmography was manufactured in which as shown in Figure 52, analog-digital conversion of signals is first carried out by means of an A-D converter 13, after which twice-differentiation is effected in digital by means of a microcomputer 14. Then, the clinical diagnosis was carried out with the result that worsening of blood flow state towards arteriosclerosis, myocardial infarction, cerebral apoplexy, subarachnoidal hemorrhage, etc. was quantitatively grasped and accurate diagnosis of presymptom of these cases became possible. In Figure 52, reference numeral 9' designates a recorder and 15 designates a monitor display.

Embodiment 3

In the embodiment 1, the distance of the valley B and valley D in the quadratic differentiated figure of the acceleration plethysmography was obtained from the figure. However, in place of such arrangement, there is proposed a cubic differentiated operation in which as shown in Figure 53, a microcomputer 17 is used to effect differentiation once again whereby obtaining the position of the valley B, valley D and so on in a numeric manner with the result that an error in reading is reduced to enhance the accuracy of diagnosis by 5%. Figure 54 (b) is a cubic differentiated figure represented on the recorder.

Embodiment 4

In recording a change of a lapse of time of the content of blood in the blood capillaries, a varying speed thereof, a varying acceleration thereof, etc. on a record paper of the pen-written oscillograph, the dial has been heretofore adjusted by hands to prevent a force-out from the width of record paper or prevent waveforms from being formed into an excessively small size, which operation is cumbersome. Therefore, an automatic gain control circuit 19 was employed as shown in Figure 55 with the result that recording became possible to be made neatly within a predetermined width to cut examination time by 25%. In Figure 55, the reference numeral 18 designates a condition amplifier.

Embodiment 5

Only one quadratic differentiated figure of the acceleration plethysmography has been illustrated in the embodiment 1. However, since the pulse wave is naturally changed somewhat in time intervals by the respiration action, this has to be compensated for. Thus, the embodiments 2 and 3 were further developed so that statistical processes such as arithmetical average of information on the corresponding maximum and minimum values of a plurality of repetitive waveforms are effected to record data. In this manner, the cause of variation due to the respiration action or the like can now be removed satisfactorily.

Embodiment 6

Since the acceleration pulse wave meter is possible to promptly provide simple and accurate diagnostic information, it is necessary to install on the street a robot doctor 16 by which people who conduct themselves an athletic remedy such as tennis, jogging or the like can easily receive treatment. To meet said need, an automatic diagnostic apparatus as shown in Figure 56 was fabricated which comprises a sensor 20 into which a hand's finger-apex is inserted, a coin mechanism 21 which receives charge and an ejector 22 which provides a card representative of diagnostic results, the aforementioned diagnostic algorithm being stored into a microcomputer. This apparatus is designed so that when a coin is deposited by a user or when a membership card with a magnetic stripe is inserted into a predetermined location 23, a power source of the apparatus is closed and then, a finger is inserted into the sensor whereby data are obtained to print out the diagnostic result. It was found from the diagnosis of the case that the apparatus can diagnose in a manner substantially equal to the inventor. With this apparatus, a patient need not to take the trouble to go to a hospital and became possible to make the control of his own health at a low cost and whenever he wished to do so.

It is noted that the apparatus can give instructions even about a proper amount of exercise, a dietary remedy and the like.

While in the above-described embodiments, the photoelectric type finger-apex pulse wave sensor has been used, pressure type or impedance type can also be used for detection. Further, parts to be detected are not limited to the finger-apex, and the method using the quadratic differentiation and cubic differentiation in accordance with the present invention may be applied to the detection device if the latter can observe a change of a lapse of time of the blood volume contained in the blood capillaries and artery system of an ear-lobe, brain or other parts of the body. Also, in case that a diagnostician is skillful and an inspection record is not necessary, the quadratic differentiated figure merely need be displayed on a Braun tube oscillograph.

As described above, in the present invention, a contrivance has been made to further analyse the conventional pulse wave curve to express it understandably. Yet the use of the present system was made possible to grasp the quality of individual's blood circulation despite the stage in which a minor study is just made, and it has been apparent that a disease possibly followed may be inferred to take necessary preventive step.

Moreover, the present invention can advantageously provide high-degree diagnosis and extensive applications as not achieved by the conventional plethysmography since the result of improvements obtained by training, running, etc. may be judged from the acceleration pulse wave curve and the apparatus can make an early discovery of disease and observe even a transition of the improvement thereof for use as a monitor.

## Claims

1. The use of a twice-differentiated plethysmographic signal to provide an evaluation of the quality of circulation of blood in a blood capillary bed between an artery and a vein of a finger-tip, said signal having been obtained by means of a plethysmograph, which is known per se and which comprises a pick-up (5) adapted to detect variations in the blood content of said finger-tip consequent upon a heart beat and to provide a first signal representative of said variations, circuit means (8) for twice differentiating said first signal with respect to time, and means for displaying or recording said twice-differentiated signal in such a manner in relation to a magnitude reference axis (Figures 48 and 49) as to indicate the relative magnitudes of at least the first and second valleys (B, D) and the second peak (C) of the waveform of said twice-differentiated signal, characterised in that said magnitudes of said valleys (B, D) and peak (C) are compared with predetermined values of said magnitudes to provide a quantitative evaluation of said blood circulation quality.

2. Automatic diagnostic apparatus comprising:

a pick-up (5) adapted to receive a finger therein, to detect a predetermined physiological condition in said finger and to provide a first signal representative of said detected physiological condition;

means utilizing a diagnostic algorithm to obtain from said first signal diagnostic data related to said predetermined physiological condition; and

means (9) for displaying said diagnostic data;

characterised in that:

said pick-up (5) is a plethysmographic pick-up (5) adapted to detect variations in the blood content of a finger-tip consequent upon a heart beat and to provide said first signal representative of said variations (known per se);

circuit means (8) is provided for twice-differentiating said first signal with respect to time (known per se);

said utilizing means comprises means for comparing the magnitude of the waveform of said twice-differentiated signal in relation to a magnitude reference axis (Figures 48 and 49), said diagnostic algorithm being utilized to compare the relative magnitudes of at least the first and second valleys (B, D) and the second peak (C) of the waveform of said twice-differentiated signal with a plurality of predetermined standard waveforms (Figure 48) to provide an evaluation of the quality of circulation of blood in a blood capillary bed between an artery and a vein of said finger-tip.

## Patentansprüche

1. Verwendung eines doppel-differenzierten plethysmographischen Signals für die Ermöglichung einer Bewertung der Güte der Blutzirkulation in einem Blutkapillargefäßbett zwischen einer Arterie und einer Vene einer Fingerspitze, wobei das Signal mittels eines an sich bekannten Plethysmographen bzw. Volumpulskurvenschreibers gewonnen wurde, der einen Abnehmer (5) zum Erfassen von Änderungen im Blutgehalt der Fingerspitze in Abhängigkeit von einem Herzschlag und zur Lieferung eines ersten, für diese Änderungen repräsentativen Signals, eine Schaltungseinheit (8) zum Doppel-Differenzieren des ersten Signals in Abhängigkeit von der Zeit und eine Einrichtung zum Anzeigen oder Aufzeichnen des doppel-differenzierten Signals in bezug auf eine Größenbezugsachse (Fig. 48 und 49) in der Weise, daß die Relativgrößen von zumindest den ersten und zweiten Kurventälern (B, D) und der zweiten Spitze (C) der Wellenform des doppel-differenzierten Signals angezeigt werden, aufweist, dadurch gekennzeichnet, daß die Größen der Kurventäler (B, D) und der Spitze (C) mit vorbestimmten Werten der Größen verglichen werden zur Ermöglichung einer quantitativen Bewertung der Blutzirkulationsgüte.

2. Automatisches Diagnosegerät, umfassend einen Abnehmer (5), der eine Fingerspitze aufzunehmen vermag, um einen vorbestimmten physiologischen Zustand im Finger zu erfassen und ein erstes, für den erfaßten physiologischen Zustand repräsentatives Signal zu lierfern,

eine einen diagnostischen Algorithmus verwendende Einrichtung, um aus dem ersten Signal auf den vorbestimmten physiologischen Zustand bezogene diagnostische Daten zu gewinnen,

(und) eine Einrichtung (9) zum Wiedergeben der diagnostischen Daten,

dadurch gekennzeichnet, daß

der Abnehmer (5) ein plethysmographischer Abnehmer (5) ist, der Änderungen im Blutgehalt (-inhalt) einer Fingerspitze in Abhängigkeit von einem Herzschlag zu erfassen und das erste, für die Änderungen repräsentative Signal zu liefern vermag (an sich bekannt),

eine Schaltungseinheit (8) zum Doppel-Differenzieren des ersten Signals in Abhängigkeit von der Zeit vorgesehen ist (an sich bekannt), (und) die den Algorithmus verwendende Einrichtung Mittel zum Vergleichen der Größe der Wellenform des doppel-differenzierten Signals in bezug auf eine Größenbezugsachse (Fig. 48 und 49) aufweist, wobei der diagnostische Algorithmus benutzbar ist

zum Vergleichen der Relativgrößen von zumindest den ersten und zweiten Kurventälern (B, D) und der zweiten Spitze (C) der Wellenform des doppel-differenzierten Signals mit einer Anzahl vorbestimmter Standardwellenformen (Fig. 48) zwecks Ermöglichung einer Bewertung der Güte der Blutzirkulation in einem Blutkapillargefäßbett zwischen einer Arterie und einer Vene der Fingerspitze.

**Revendications**

1. L'utilisation d'un signal pléthysmographique différentié deux fois pour fournir une évaluation de la qualité de la circulation du sang dans un réseau capillaire entre une artère et une veine d'un bout de doigt, ce signal ayant été obtenu au moyen d'un pléthysmographe, qui est connu en luimême et qui comprend un capteur (5) conçu de façon à détecter des variations de la quantité de sang contenue dans le bout du doigt sous l'effet d'un battement du coeur, et à fournir un premier signal représentatif de ces variations, un circuit (8) destiné à différentier deux fois le premier signal par rapport au temps, et des moyens destinés à visualiser ou enregistrer le signal différentié deux fois, de manière à indiquer, en relation avec un axe de référence d'amplitude (figures 48 et 49), les amplitudes relatives des premier et second creux (B, D) et de la seconde crête (C), au moins, de la forme d'onde du signal différentié deux fois, caractérisée en ce qu'on compare les amplitudes des creux (B, D) et de la crête (C) avec des valeurs prédéterminées de ces amplitudes, pour fournir une évaluation quantitative de la qualité de la circulation du sang.

2. Appareil de diagnostic automatique comprenant:

un capteur (5) conçu de façon à recevoir un doigt à l'intérieur, pour détecter une condition physiologique prédéterminée dans ce doigt, et pour fournir un premier signal représentatif de la condition physiologique détectée;

des moyens utilisant un algorithme de diagnostic pour obtenir à partir de ce premier signal des données de diagnostic liées à la condition physiologique prédéterminée; et

des moyens (9) destinés à visualiser les données de diagnostic;

caractérisé en ce que:

le capteur (5) est un capteur pléthysmographique (5) conçu de façon à détecter des variations de la quantité de sang contenue dans un bout de doigt sous l'effet d'un battement du coeur, et à fournir le premier signal représentatif de ces variations (ce capteur est connu en lui-même);

un circuit (8) est prévu pour différentier deux fois le premier signal par rapport au temps (ce circuit est connu en lui-même);

les moyens d'utilisation comprennent des moyens destinés à comparer l'amplitude de la forme d'onde du signal différentié deux fois, en relation avec un axe de référence d'amplitude (figures 48 et 49), et l'algorithme de diagnostic est utilisé pour comparer les amplitudes relatives des premier et second creux (B, D) et de la première crête (C), au moins, de la forme d'onde du signal différentié deux fois, avec un ensemble de formes d'ondes de type standard prédéterminées (figure 48), pour fournir une évaluation de la qualité de la circulation du sang dans un réseau capillaire entre une artère et une veine du bout de doigt.

FIG. 1

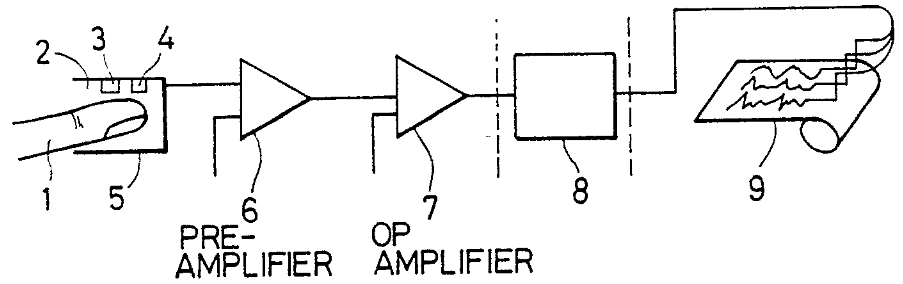


FIG. 2

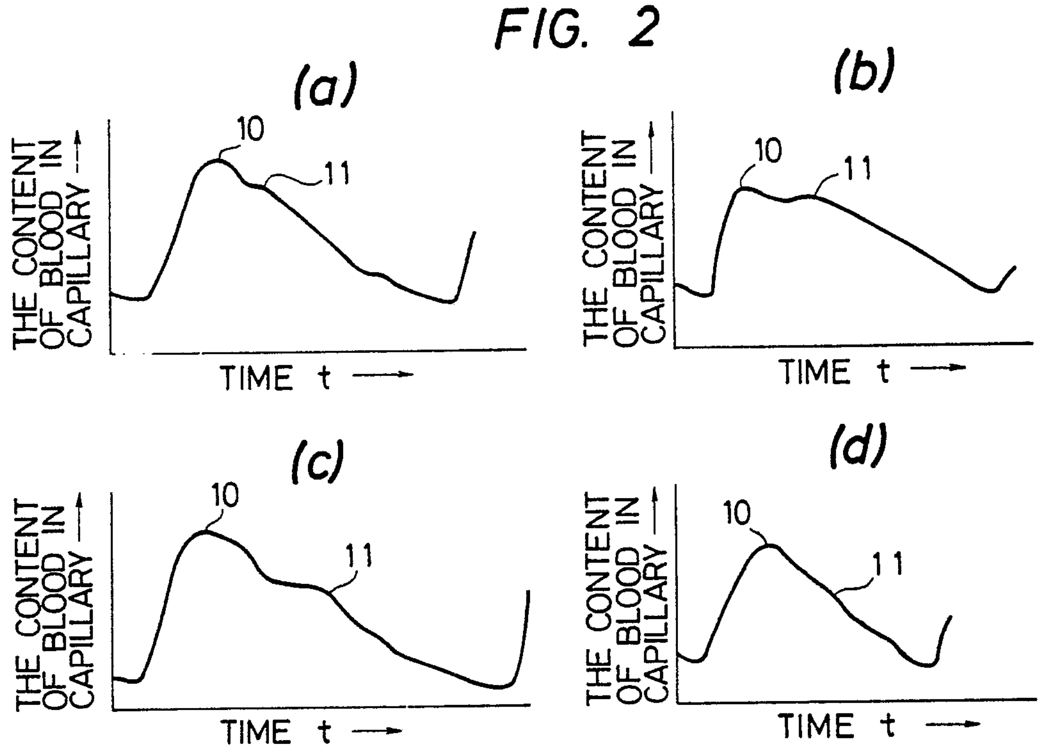


FIG. 3

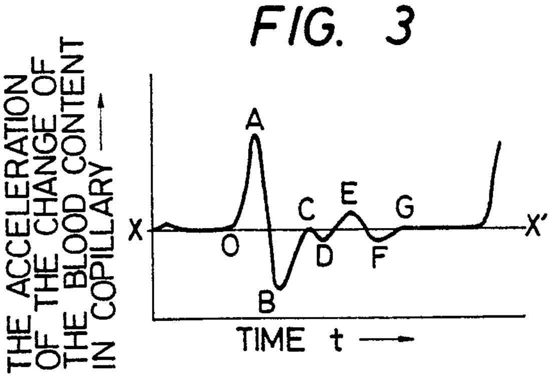


FIG. 4

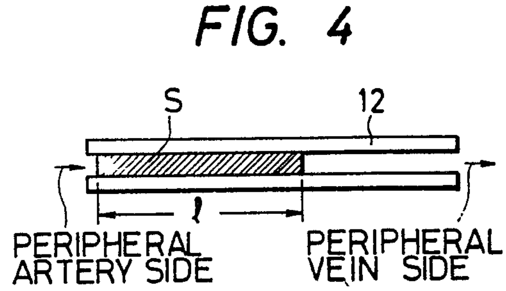

FIG. 5 73♂ FIG. 6 64♂ FIG. 7 40♂ FIG. 8 31♀ FIG. 9 38♀ FIG. 10 26♂ FIG. 11 36♂

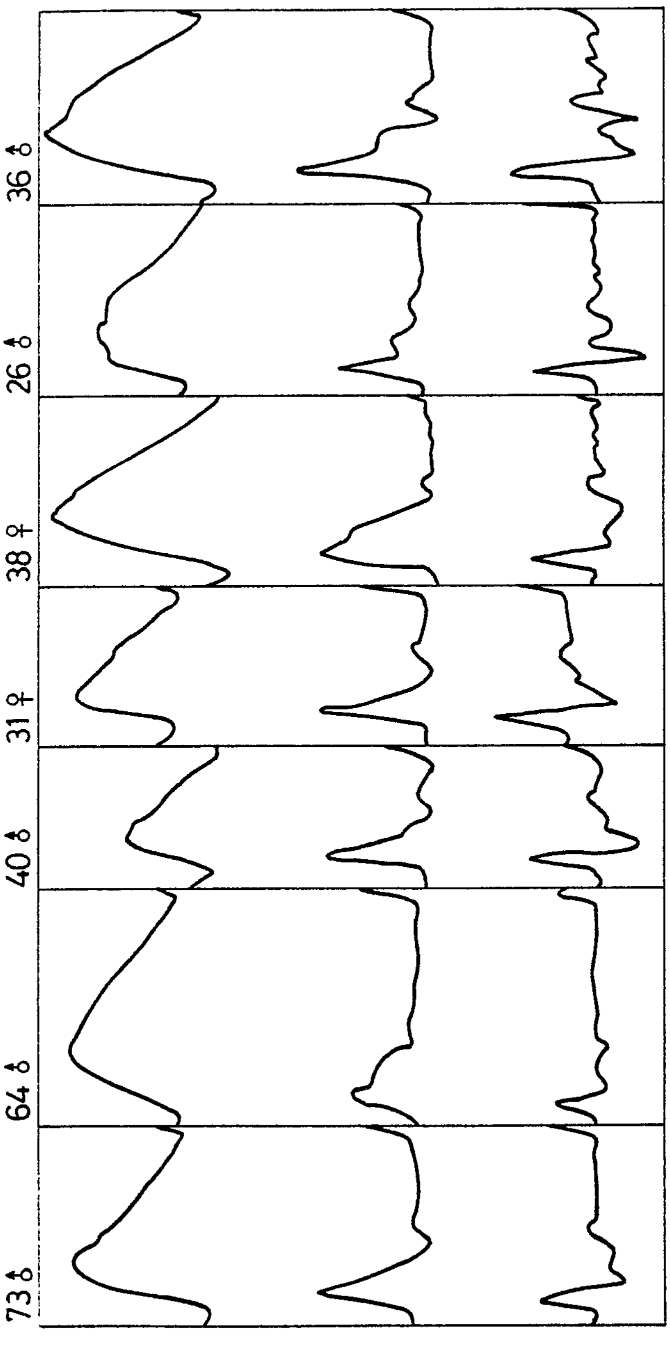

FIG. 16

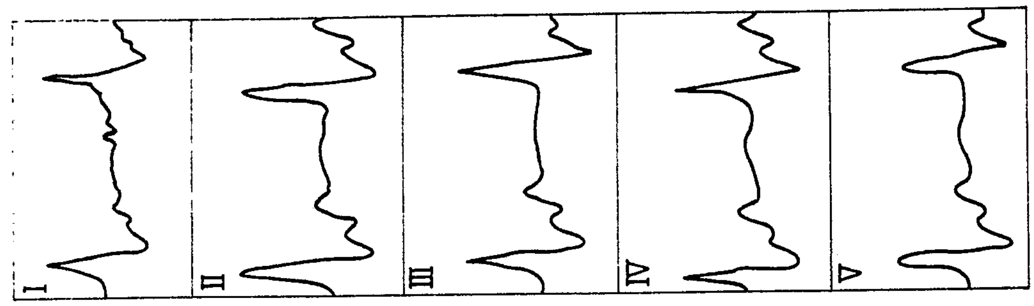


FIG. 12 FIG. 13 FIG. 14 FIG. 15

52 39 26 23

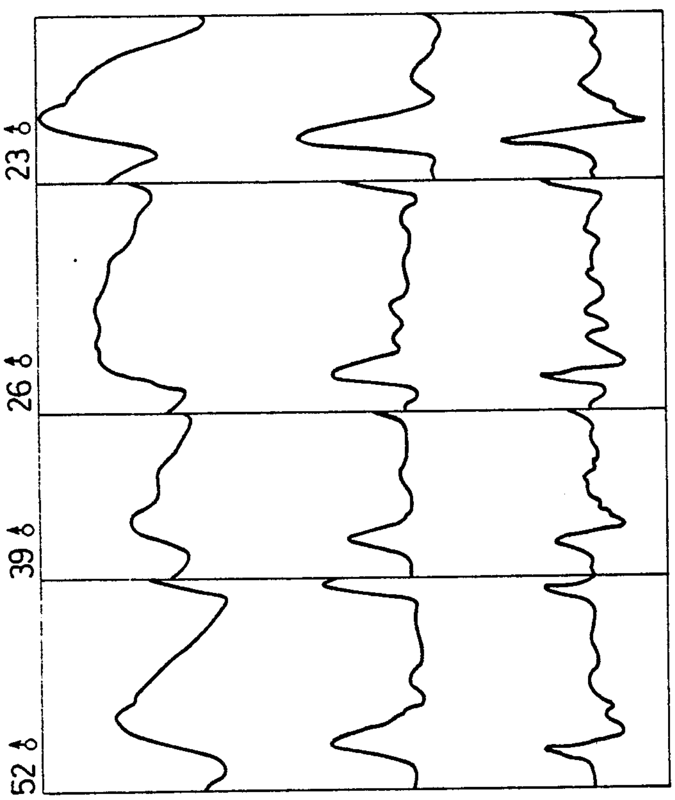

FIG. 17 FIG. 18 FIG. 19 FIG. 20 FIG. 21 FIG. 22 FIG. 23 FIG. 24

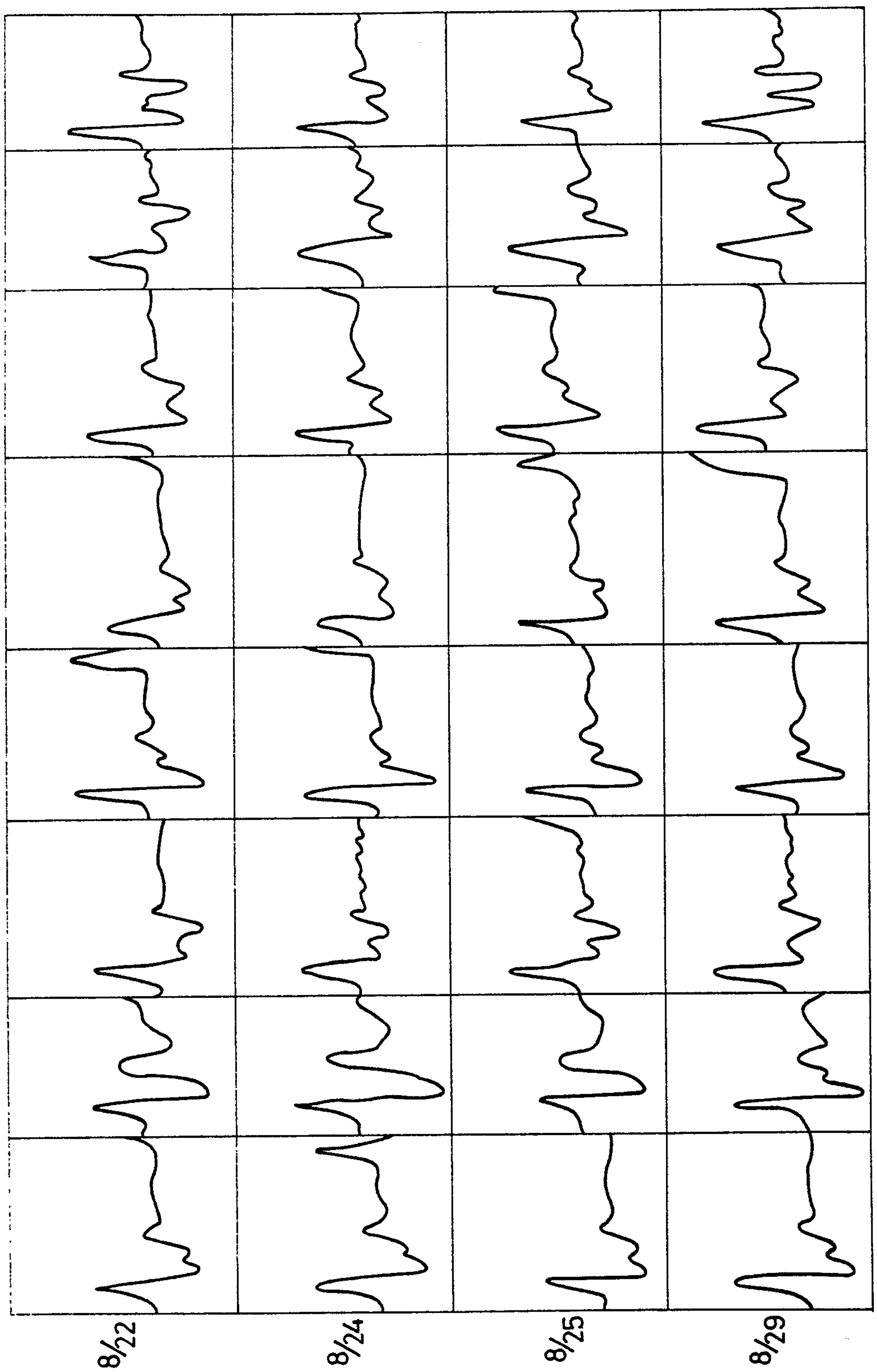

8/22 8/24 8/25 8/29

FIG. 25 FIG. 26 FIG. 27 FIG. 28 FIG. 29 FIG. 30 FIG. 31

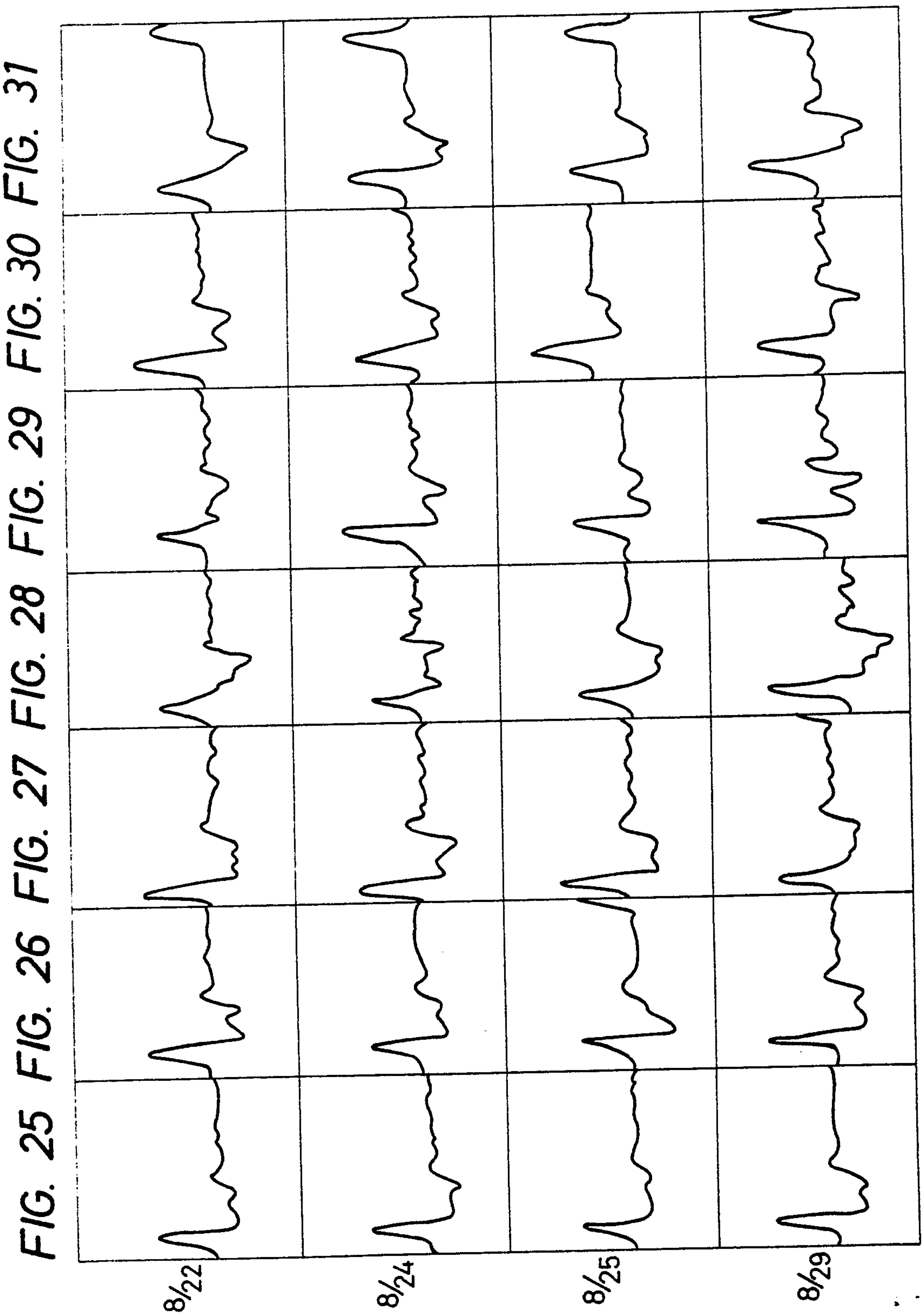

FIG. 32 FIG. 33 FIG. 34 FIG. 35 FIG. 36 FIG. 37 FIG. 38

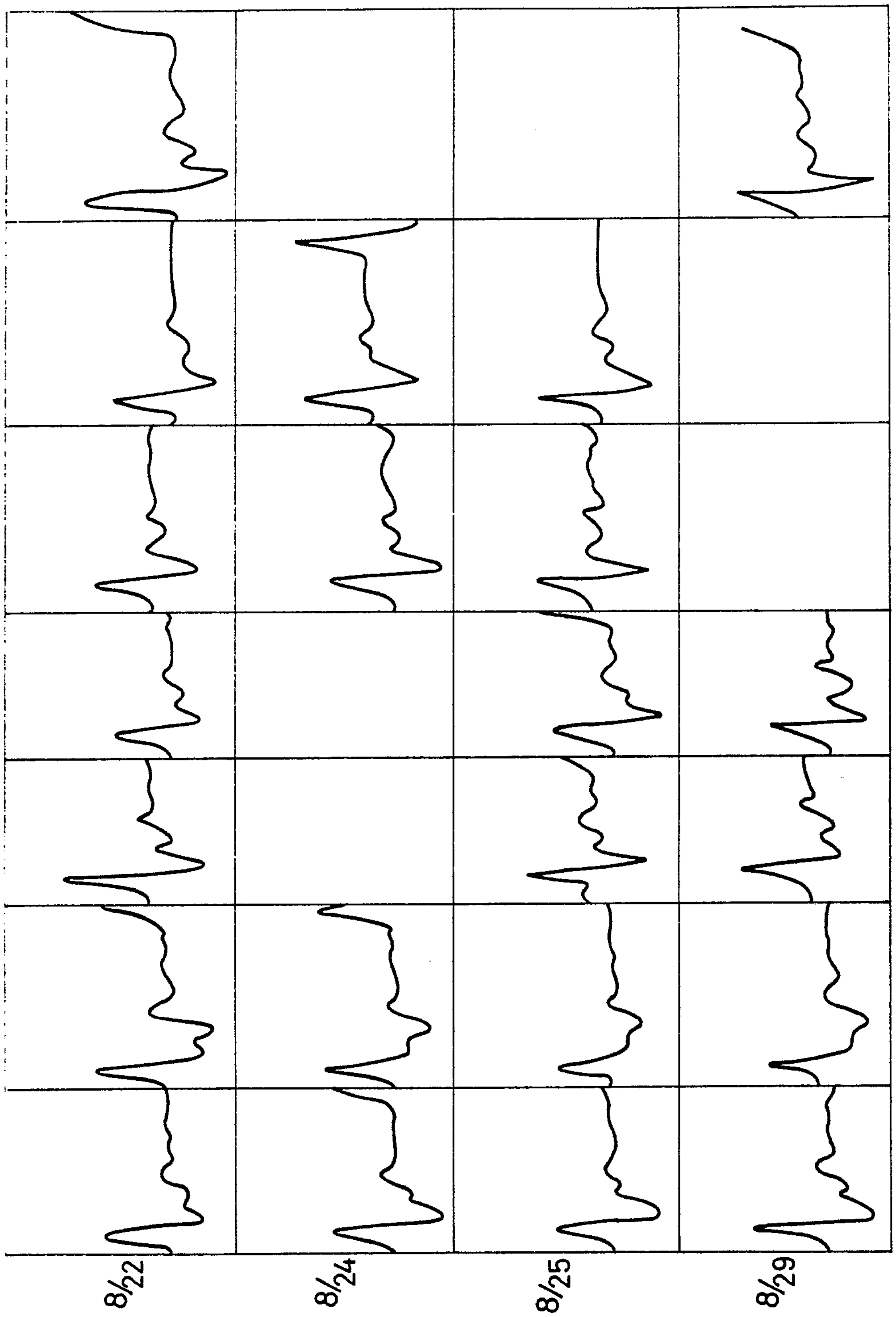

FIG. 39 FIG. 40 FIG. 41 FIG. 42 FIG. 43

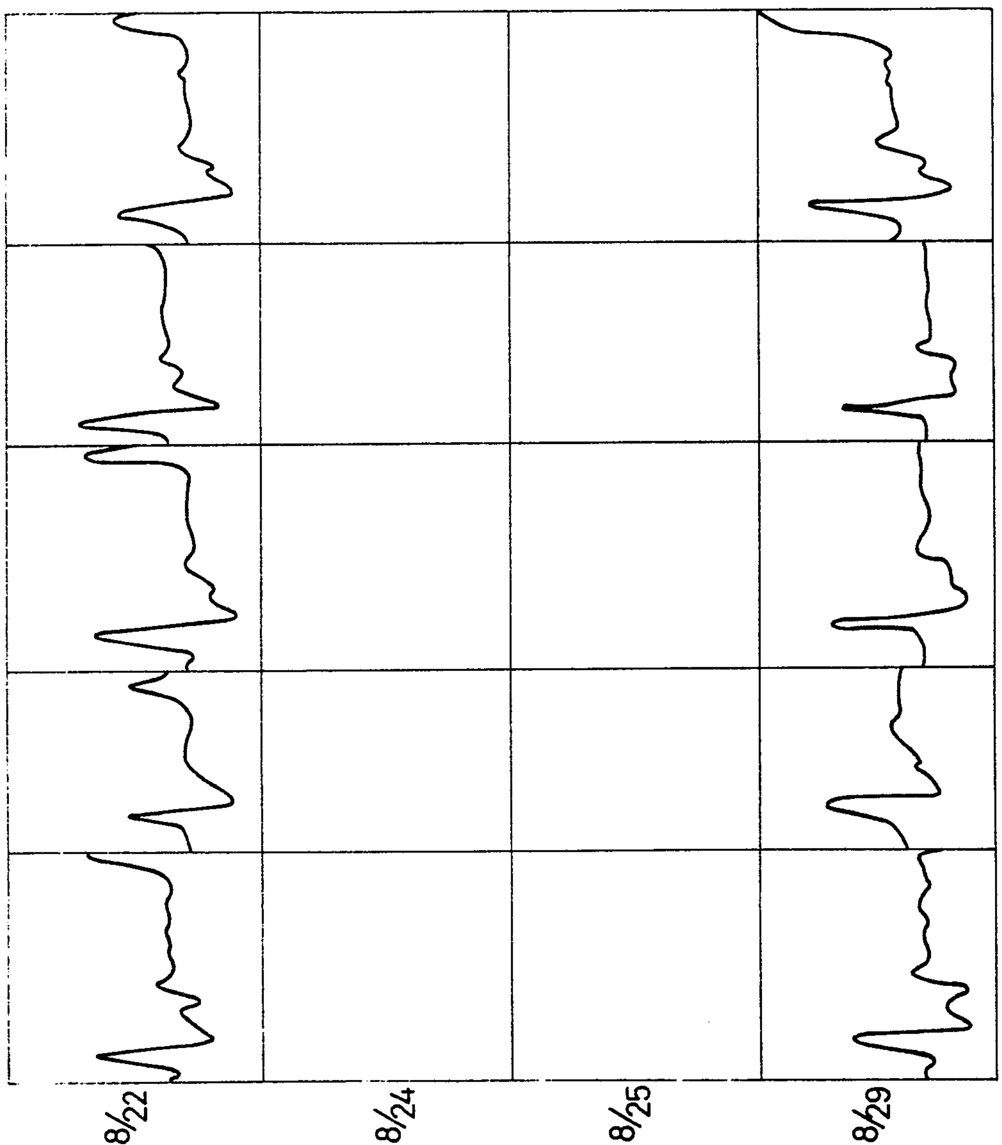

8/22

8/24

8/25

8/29

FIG. 44

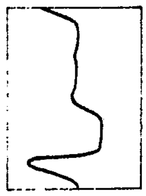

FIG. 45

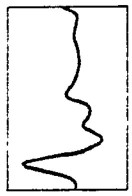

FIG. 46

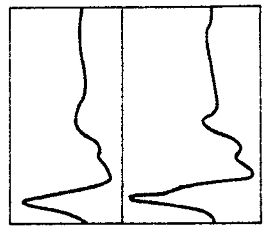

(a)

(b)

FIG. 47

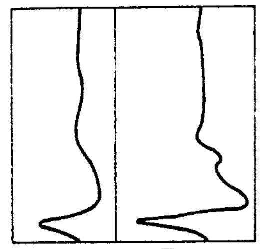

(a)

(b)

FIG. 48

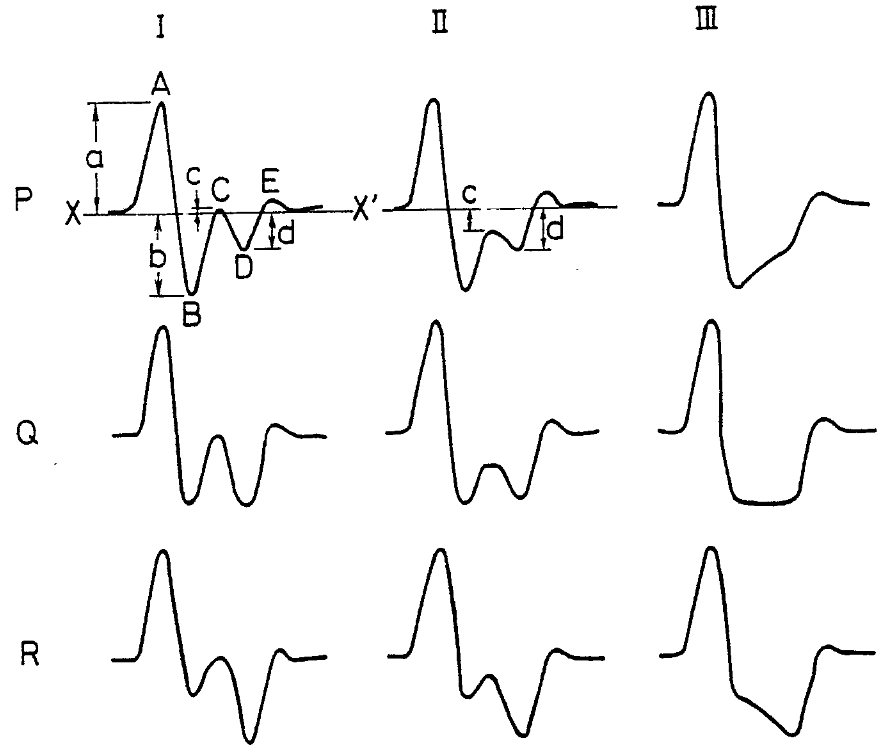


FIG. 49

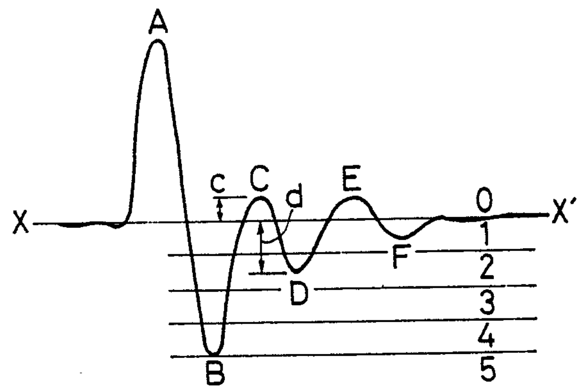

FIG. 50

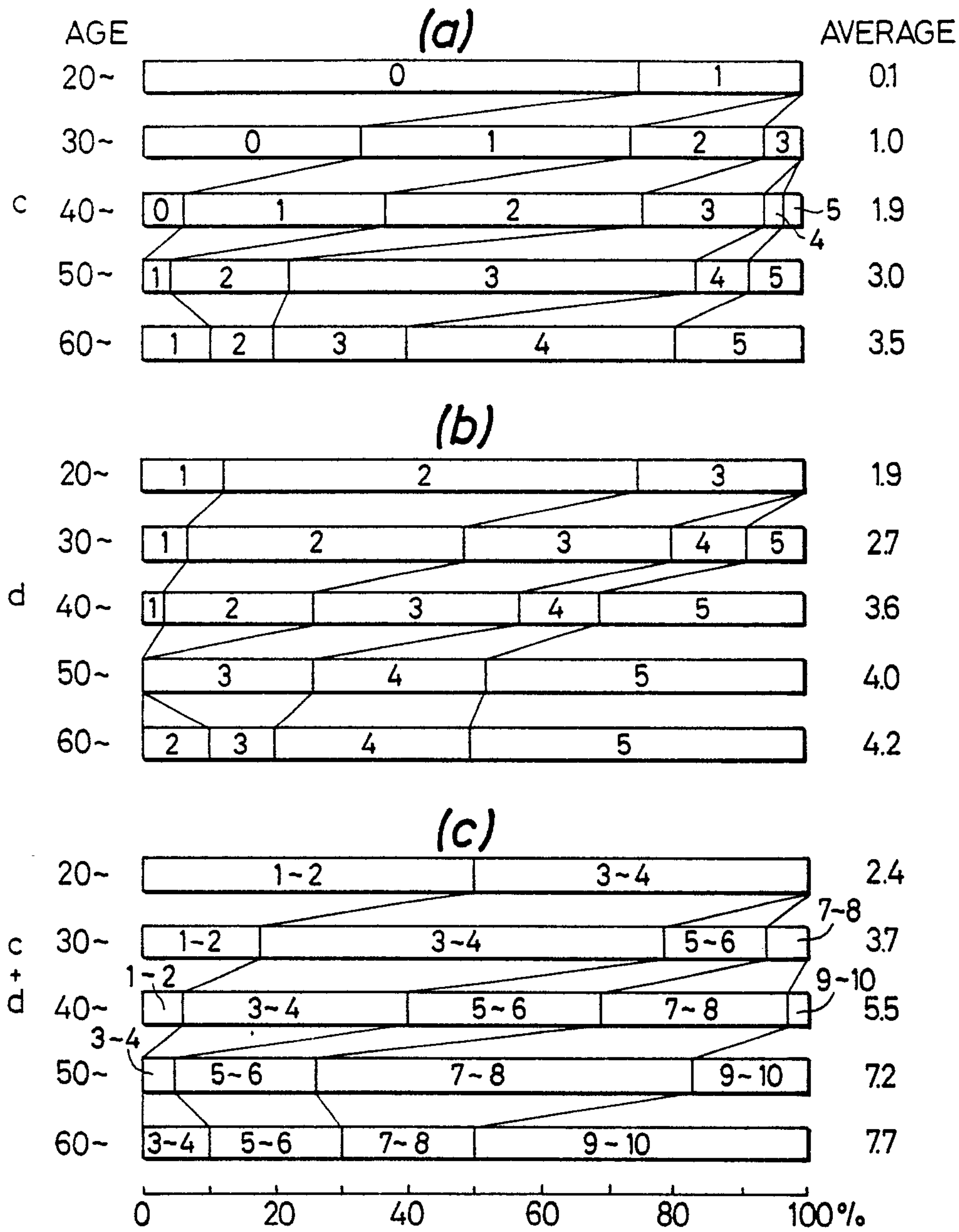
(a)
AGE
AVERAGE
20~
0
1
0.1
30~
0
1
2
3
1.0
c
40~
0
1
2
3
5
4
1.9
50~
1
2
3
4
5
3.0
60~
1
2
3
4
5
3.5
(b)
20~
1
2
3
1.9
30~
1
2
3
4
5
2.7
d
40~
1
2
3
4
5
3.6
50~
3
4
5
4.0
60~
2
3
4
5
4.2
(c)
20~
1~2
3~4
2.4
c + d
30~
1~2
3~4
5~6
7~8
3.7
40~
1~2
3~4
5~6
7~8
9~10
5.5
50~
3~4
5~6
7~8
9~10
7.2
60~
3~4
5~6
7~8
9~10
7.7
0
20
40
60
80
100%

FIG. 51

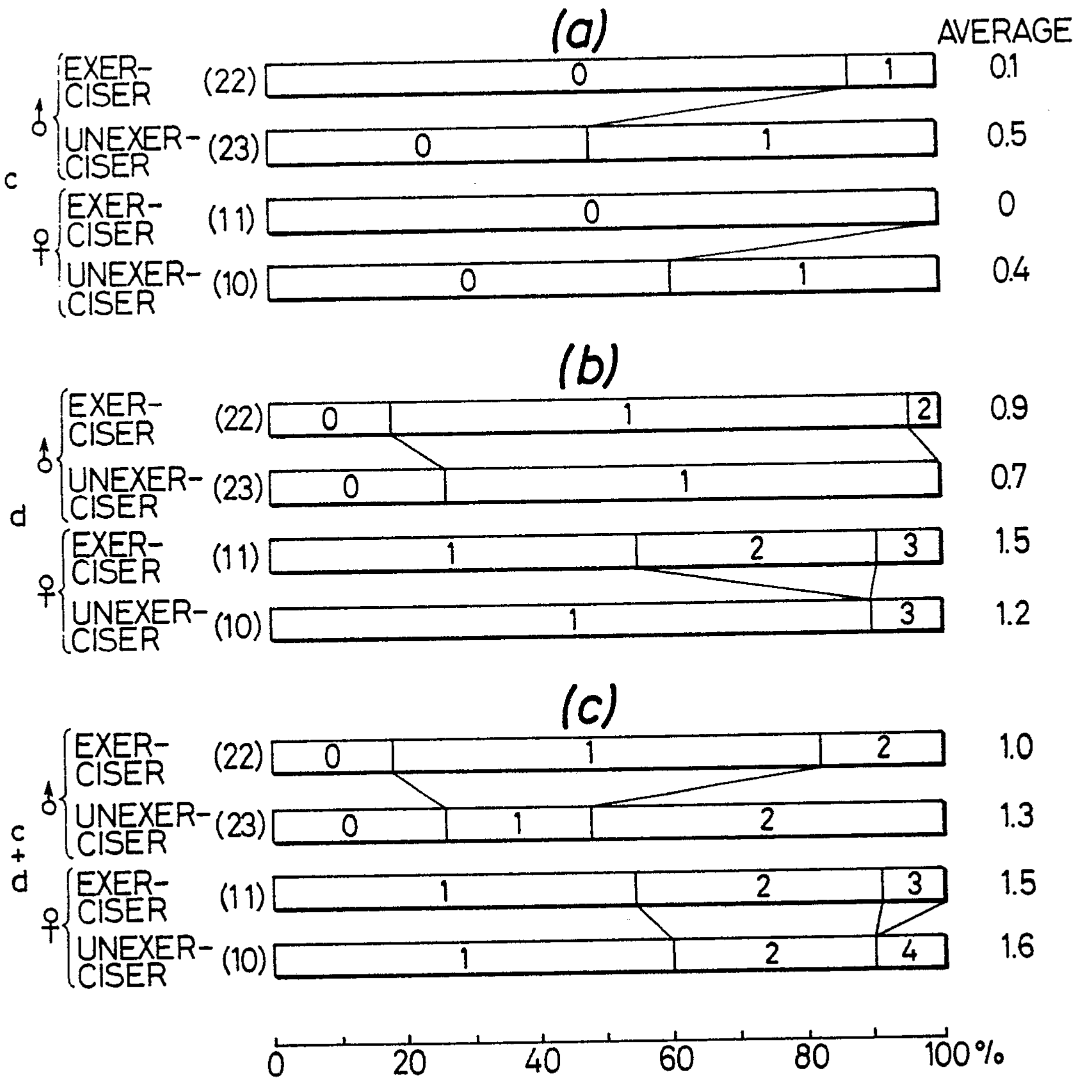
(a)
AVERAGE
♂
EXER-CISER (22) 0 1 0.1
UNEXER-CISER (23) 0 1 0.5
c
♀
EXER-CISER (11) 0 0
UNEXER-CISER (10) 0 1 0.4
(b)
♂
EXER-CISER (22) 0 1 2 0.9
UNEXER-CISER (23) 0 1 0.7
d
♀
EXER-CISER (11) 1 2 3 1.5
UNEXER-CISER (10) 1 3 1.2
(c)
♂
EXER-CISER (22) 0 1 2 1.0
UNEXER-CISER (23) 0 1 2 1.3
c + d
♀
EXER-CISER (11) 1 2 3 1.5
UNEXER-CISER (10) 1 2 4 1.6
0 20 40 60 80 100%

FIG. 52

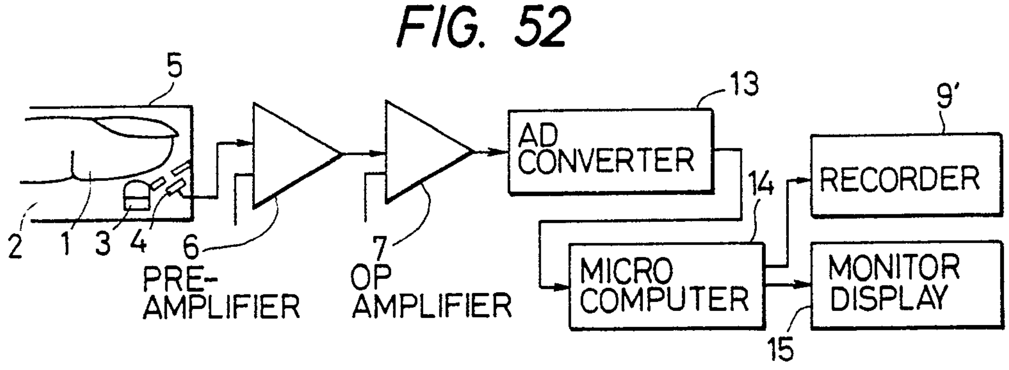


FIG. 53

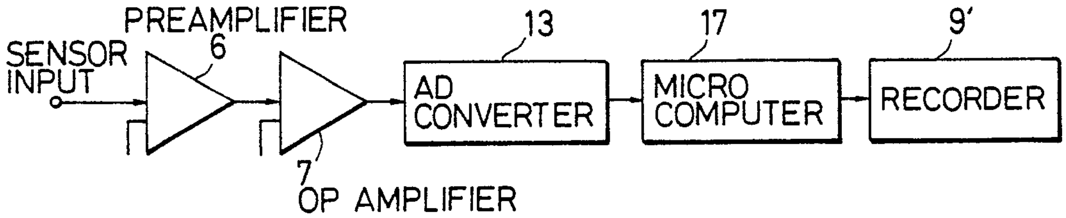


FIG. 54

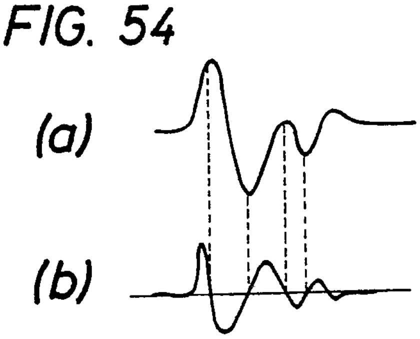


FIG. 56

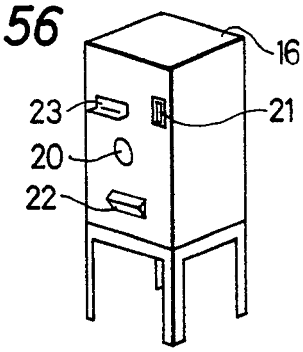


FIG. 55